# EUROPÄISCHE PATENTANMELDUNG

(11) **EP 1 316 300 A1**
(43) Veröffentlichungstag der Anmeldung: **04.06.2003**
(21) Anmeldenummer: 02025778.8
(22) Anmeldetag: 16.11.2002
(51) Int. Cl.: A61K 7/42

(54) **Verwendung von mehrphasigen Mitteln zum Verschäumen aus Schaumspendern**

(30) Priorität: 30.11.2001 DE 10159002
(71) Anmelder: Clariant GmbH, 65929 Frankfurt am Main (DE)
(72) Erfinder: Henning, Torsten, Dr., 65812 Bad Soden (DE)
(74) Vertreter: Paczkowski, Marcus, Dr.

(57) **Zusammenfassung**

Die Erfindung betrifft die Verwendung von mehrphasigen Mitteln, enthaltend
a) mindestens eine Ölphase und
b) mindestens eine wässrige Phase,
zum Verschäumen aus Schaumspendern.

Bevorzugt handelt es sich bei den Mitteln um kosmetische Mittel, besonders bevorzugt um Sonnenschutzmittel.

## Beschreibung

Die DE 199 55 375 beschreibt verschäumbare kosmetische Sonnenschutzmittel in Form von O/W Emulsionen. Zur Vermittlung von Ölphase und wässriger Phase enthalten die Emulsionen Emulgatoren. Die Mittel werden aus speziellen Schaumspendern verschäumt und lassen sich so leicht und gleichmäßig auf der Haut verteilen.

Überraschenderweise wurde nun gefunden, dass sich mehrphasige Mittel, enthaltend Ölphasen und wässrige Phasen, besonders gut aus Schaumspendern verschäumen lassen und einen verbesserten Schaum bilden. Bei kosmetischen Anwendungen werden das Hautgefühl und die Verteilbarkeit auf der Haut deutlich verbessert. Gegenüber Emulsionen haben die Mittel den Vorteil, dass sie frei sein können von Emulgatoren, die teilweise als physiologisch bedenklich angesehen werden.

Gegenstand der Erfindung ist demnach die Verwendung von mehrphasigen Mitteln, enthaltend
a) mindestens eine Ölphase und
b) mindestens eine wässrige Phase,
zum Verschäumen aus Schaumspendern.

Unter mehrphasigen Mitteln sind solche zu verstehen, bei denen die Ölphasen und die wässrigen Phasen jeweils als makroskopische Phasen getrennt voneinander vorliegen. Bei den erfindungsgemäßen Mitteln handelt es sich also nicht um Emulsionen, die zur Vermittlung von Ölphase und wässriger Phase regelmäßig Emulgatoren enthalten.

Bevorzugt handelt es sich bei den Mitteln um Zweiphasensysteme, die aus einer Ölphase und einer wässrigen Phase bestehen.

Der Anteil der Ölphasen beträgt, bezogen auf die fertigen Mittel, bevorzugt 1 bis 50 Gew.-%, besonders bevorzugt 3 bis 40 Gew.-%.
Der Anteil der wässrigen Phasen beträgt, bezogen auf die fertigen Mittel, bevorzugt 50 bis 99 Gew.-%, besonders bevorzugt 60 bis 97 Gew.-%.

Als Ölphasen eignen sich bevorzugt Guerbetalkohole, Fettsäureester, Ester von Fettalkoholen, Triglyceride, pflanzliche Öle, aliphatische und cycloaliphatische Kohlenwasserstoffe, Ether, Siliconöle und/oder Sonnenschutzverbindungen.
Die Ölphase kann zusätzlich auch Parfümöle enthalten.
Besonders bevorzugt als Ölphase sind Guerbetalkohole mit 6 bis 18, vorzugsweise 8 bis 10 Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen; Ester von linearen (C₆-C₁₈)-Fettsäuren mit verzweigten Alkoholen, bevorzugt 2-Ethylhexanol; Ester von linearen und verzweigten (C₆-C₁₃)-Fettsäuren mit mehrwertigen Alkoholen, bevorzugt Dimerdiol und Trimerdiol und/oder Guerbetalkoholen mit 6 bis 18 Kohlenstoffatomen; Triglyceride auf Basis von (C₆-C₁₀)-Fettsäuren; Avocadoöl; Mandelöl; Olivenöl; Jojobaöl; Dialkylether; Hexadecan; Cyclohexan; substituierte Cyclohexane;
Als Siliconöle besonders bevorzugt sind Cyclomethicon, Dimethicon, Alkylmethicone, bevorzugt Caprylylmethicon, Dialkylmethicone, bevorzugt Stearyl Dimethicon, (C₂₀-C₂₄)-Alkyl Dimethicone und/oder (C₂₄-C₂₈)-Alkyl Dimethicone, Caprylyltrimethicon, Phenyltrimethicon, Dimethylpolysiloxan, Methylphenylpolysiloxane, cyclische Silicone, sowie amino-, fettsäure-, alkohol-, polyether-, epoxy-, fluor- und/oder alkylmodifizierte Siliconverbindungen, sowie Polyalkylsiloxane, Polyalkylarylsiloxane und Polyethersiloxan-Copolymere, wie in US 5,104,645 und den darin zitierten Schriften beschrieben, die bei Raumtemperatur sowohl flüssig als auch harzförmig vorliegen können.

Als Ölphase-bildende Sonnenschutzverbindungen besonders bevorzugt sind 2-Ethylhexyl-p-Methoxycinnamate, Ethylhexyl Salicylate, Octocrylene, Oxybenzone/Benzophenone-3, Benzophenone-4, Benzophenone-5, Ethylhexyl N,N-Dimethylaminobenzoate, 4-Aminobenzoesäure/PABA, Ethylhexyl Dimethyl PABA, Phenylbenzimidazole Sulfonic Acid, Homomenthyl. Salicylate, Homosalate, Isoamyl Methoxycinnamate, 4-Methylbenzylidene Camphor, 3-Benzylidene Camphor, Benzene-1,4-[bis(3-methylidenecamphormethylsuphonic acid)], Camphor Benzalkonium Methosulfate, Butyl Methoxydibenzoylmethane, Polyacrylamidomethyl Benzylidene Camphor, PEG-25 PABA, Ethylhexyl Triazone, Drometrizole Trisiloxane, Methylene Bis-Benztriazolyl Tetramethylbutylphenol, Dioctyl Butamido Triazone, Bis-Ethylhexyloxyphenol Methoxyphenol Triazine und Mischungen derselben.

Bei den wässrigen Phasen handelt es sich bevorzugt um wässrige Lösungen enthaltend schaumverstärkende Tenside, Alkohole, Salze, Polymere und/oder weitere pflegende Zusätze.

In einer besonders bevorzugten Ausführungsform enthalten die wässrigen Phasen schaumverstärkende Tenside, die die Schaumbildung und die Schaumqualität deutlich verbessern.
Wesentlich für den Einsatz der Tenside ist dabei, dass die Tenside so gewählt werden oder in einer solchen Menge zugesetzt werden, dass der Mehrphasencharakter der Mittel erhalten bleibt. D.h. die Tenside dürfen für das jeweilige Öl/ Wasser System nicht als Emulgator wirken, was zur Bildung einer Emulsion führen würde. Nach Griffin (K. Shinoda et al., Emulsions and Solubilisation, Wiley, New York, 1986, Seite 72) ist bekannt, dass nur solche Tenside als Emulgatoren fungieren, die einen HLB-Wert (hydrophilic lipophilic balance) von kleiner als 18 zeigen. Die schaumverstärkenden Tenside besitzen demnach bevorzugt einen HLB-Wert von größer 18, besonders bevorzugt größer 20, insbesondere bevorzugt von 20 bis 40.
Überraschend wurde gefunden, dass anionische Tenside besonders gut als schaumverstärkende Tenside geeignet sind.
Besonders bevorzugt als schaumverstärkende Tenside sind Acylglutamate, (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate und/oder -glycinate.
Insbesondere bevorzugt als Tenside sind Acylglutamate, bevorzugt Natrium Lauroylglutamat und/oder Natrium Cocoylglutamat.
Bevorzugt werden die Tenside in Form ihrer wasserlöslichen oder in Wasser dispergierbaren Salze benutzt, bevorzugt in Form der Natrium-, Kalium-, Magnesium-, Ammonium-, Mono-, Di- und Triethanolammonium- sowie analogen Alkylammonium-Salze.
Der Anteil der schaumverstärkenden Tenside in den wässrigen Phasen beträgt, bezogen auf die wässrigen Phasen, bevorzugt 0,1 bis 50 Gew.-%, besonders bevorzugt 0,1 bis 20 Gew.-%, insbesondere bevorzugt 0,3 bis 15 Gew.-%.

Überraschend wurde gefunden, dass sich die erfindungsgemäßen Mittel besonders vorteilhaft verschäumen lassen, wenn die Ölphasen und/oder die wässrigen Phasen, besonders bevorzugt die Ölphasen und die wässrigen Phasen, niederviskos vorliegen.
Die Viskositäten der Ölphasen betragen bevorzugt 1 bis 1000 mPas, besonders bevorzugt 10 bis 500 mPas.
Die Viskositäten der wässrigen Phasen betragen bevorzugt 1 bis 1000 mPas, besonders bevorzugt von 10 bis 500 mPas.

Weiterhin wurde gefunden, dass sich der Zusatz von 0,01 - 5 Gew.-% an Elektrolyten und/oder ein- und/oder mehrwertigen Alkoholen, jeweils bezogen auf die wässrigen Phasen, besonders vorteilhaft auf die Verschäumbarkeit auswirkt.
Als Elektrolyte eignen sich bevorzugt Salze von Alkalimetallen und/oder Erdalkalimetallen, bevorzugt Natriumchlorid und/oder Kaliumchlorid.
Als Alkohole eignen sich bevorzugt Propylenglykol, Isopropanol, Ethanol und/oder Polyethylenglykole. Bevorzugt sind Polyethylenglykole mit einer relativen Molekülmasse zwischen 200 und 600 g/mol, bevorzugt in Mengen von 0,1 bis 45 Gew.-% bezogen auf die wässrige Phase.

Bevorzugt handelt es sich bei den Mitteln um kosmetische Mittel, besonders bevorzugt um Hautpflegemittel. Die Hautpflegemittel können wie Emulsionen angewendet werden. Insbesondere eignen sich die Mittel als Sonnenschutzmittel, bevorzugt als Sonnenschutzschäume. Im Falle der Sonnenschutzmittel hat es sich als besonders vorteilhaft erwiesen, wenn die darin enthaltenen Sonnenschutzverbindungen selber oder zumindest teilweise die Ölphase bilden.

Als Hilfs- und Zusatzstoffe können die kosmetischen Mittel UV-Schutzmittel, Selbstbräunungsmittel, Verdickungsmittel, Überfettungsmittel, Trägermaterialien, Siliconverbindungen, hydrophile Komponenten, Fette, Wachse, biogene Wirkstoffe, Antioxidantien, Hydrotrope, Kationpolymere, Glycerin, Konservierungsmittel, Dispergiermittel, Eiweißderivate wie Gelatine, Collagenhydrolysate, Polypeptide auf natürlicher und synthetischer Basis, Fettalkohole, deodorierende Mittel, Stoffe mit keratolytischer und keratoplastischer Wirkung, Enzyme und Trägersubstanzen, feuchtigkeitsspendende Stoffe, antimikrobiell wirkende Agentien, Farb- und/oder Duftstoffe enthalten.

Als Selbstbräunungsmittel bevorzugt sind Dihydroxyacetone.

Als Überfettungsmittel eignen sich bevorzugt Fettsäurealkanolamide, die gleichzeitig als Schaumstabilisatoren dienen.

Typische Beispiele für Fette sind Glyceride, als Wachse kommen u.a. Bienenwachs, Paraffinwachse oder Mikrowachse, gegebenenfalls in Kombination mit hydrophilen Wachsen, z.B. Cetylstearylalkohol, in Frage.

Als hydrophile Komponenten eignen sich bevorzugt die sogenannten "Soil Release Polymere", insbesondere Oligoester, erhalten durch Polykondensation von 40 bis 52 Mol-%, vorzugsweise 45 bis 50 Mol-%, einer oder mehrerer Dicarbonsäuren oder deren Ester mit 10 bis 40 Mol-%, vorzugsweise 20 bis 35 Mol-% Ethylenglykol und/oder Propylenglykol, 3 bis 20 Mol-%, vorzugsweise 10 bis 15 Mol-% Polyethylenglykol, 0 bis 10 Mol-% eines wasserlöslichen Anlagerungsproduktes von 5 bis 80 mol eines Alkylenoxids an 1 mol (C₁-C₂₄)-Alkohole, (C₆-C₁₈)-Alkylphenole oder (C₈-C₂₄)-Alkylamine oder 0 bis 10 Mol-% eines oder mehrerer Polyole mit 3 bis 6 Hydroxylgruppen.

Als fungizide Wirkstoffe können Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion, und/oder Octopirox eingesetzt werden.

Als pflegende Stoffe können Allantoin und Bisabolol, bevorzugt in Mengen von 0,0001 bis 10 Gew.-%, eingesetzt werden.

Als Antioxidantien eigenen sich bevorzugt Superoxid-Dismutase, Tocopherol (Vitamin E) und Ascorbinsäure (Vitamin C) und/oder deren Derivate.

Als kationische Polymere sind bevorzugt kationische Cellulosederivate, kationische Stärke, Copolymere von Diallylammoniumsalzen und Acrylamiden, quaternierte Vinylpyrrolidon/Vinylimidazol-Polymere, Kondensationsprodukte von Polyglykolen und Aminen, quaternierte Kollagenpolypeptide, quaternierte Weizenpolypeptide, Polyethylenimine, kationische Siliconpolymere, wie z.B. Amidomethicone, Copolymere aus Adipinsäure und Dimethylaminohydroxypropyldiethylentriamin, Polyaminopolyamide und/oder kationische Chitinderivate wie beispielsweise Chitosan.

Die erfindungsgemäßen Zubereitungen können mit konventionellen Ceramiden, Pseudoceramiden, Fettsäure-N-alkylpolyhydroxyalkylamide, Cholesterin, Cholesterinfettsäureestern, Fettsäuren, Triglyceriden und/oder Cerebrosiden abgemischt werden.

Als feuchtigkeitsspendende Substanzen stehen bevorzugt Glycerin und/oder Sorbitol zu Verfügung, die bevorzugt in Mengen von 0,1 bis 50 Gew.-% eingesetzt werden.

Die Sonnenschutzmittel können eine oder mehrere Sonnenschutzverbindungen, die UV-Strahlung streuen, reflektieren oder absorbieren können, enthalten. Dabei können die Sonnenschutzverbindungen, wie schon erwähnt, auch gleichzeitig als Ölphase dienen.

Bevorzugt als Sonnenschutzverbindungen sind 2-Ethylhexyl-p-Methoxycinnamate, Ethylhexyl Salicylate, Octocrylene, Oxybenzone/Benzophenone-3, Benzophenone-4, Benzophenone-5, Ethylhexyl N,N-Dimethylaminobenzoate, 4-Aminobenzoesäure/PABA, Ethylhexyl Dimethyl PABA, Phenylbenzimidazole Sulfonic Acid, Homomenthyl Salicylate, Homosalate, Isoamyl Methoxycinnamate, 4-Methylbenzylidene Camphor, 3-Benzylidene Camphor, Benzene-1,4[bis(3-methylidenecamphormethylsuphonic acid)], Camphor Benzalkonium Methosulfate, Phenylbenzimidazole Sulfonic Acid, Terephtalidene Dicamphor Sulfonic Acid, Butyl Methoxydibenzoylmethane, Benzylidene Camphor Sulfonic Acid, Polyacrylamidomethyl Benzylidene Camphor, PEG-25 PABA, Ethylhexyl Triazone, Drometrizole Trisiloxane, Methylene Bis-Benztriazolyl Tetramethylbutylphenol, Dioctyl Butamido Triazone, Disodium Phenyl Dibenzimidazole Tetrasulfonate, Bis-Ethylhexyloxyphenol Methoxyphenol Triazine, 4-lsopropylbenzylsalicylat, Terephthalydene Dicamphor Sulfonic Acid und Mischungen derselben.
Als Pigmente/Mikropigmente können oberflächenbehandeltes oder unbehandeltes Titandioxid, Eisenoxid und/oder Zinkoxid und Mischungen daraus eingesetzt werden.

Die Herstellung der Mittel erfolgt bevorzugt derart, dass man die Ölkomponenten und die wässrigen Komponenten miteinander mischt, abfüllt und separieren lässt.

Gegenstand der Erfindung ist auch ein Verfahren zur Herstellung von Schaum, dadurch gekennzeichnet, dass man ein mehrphasiges Mittel, enthaltend
a) mindestens eine Ölphase und
b) mindestens eine wässrige Phase
mittels eines Schaumspenders verschäumt.

Ebenfalls Gegenstand der Erfindung sind Schaumspender, enthaltend mehrphasige Mittel, die
a) mindestens eine Ölphase und
b) mindestens eine wässrige Phase
enthalten.

Im Falle des Verfahren und der Schaumspender handelt es sich bei den Mitteln bevorzugt um Zweiphasensysteme, die aus einer Ölphase und einer wässrigen Phase bestehen. Der Anteil der Ölphase beträgt, bezogen auf die fertigen Mittel, bevorzugt 1 bis 50 Gew.-%, besonders bevorzugt 3 bis 40 Gew.-%. Der Anteil der wässrigen Phase beträgt, bezogen auf die fertigen Mittel, bevorzugt 50 bis 99 Gew.-%, besonders bevorzugt 60 bis 97 Gew.-%. Als Ölphase eignen sich bevorzugt die bereits beschriebenen Verbindungen. Bevorzugt enthält die wässrige Phase mindestens eines der bereits beschriebenen schaumverstärkenden Tenside.

Das Verfahren zur Schaumherstellung ist nicht auf die Erzeugung kosmetischer Schäume beschränkt, sondern eignet sich allgemein zur Schaumherstellung, auch für den industriellen Maßstab.

Unter Schaumspendern sind alle Apparaturen zu verstehen, die zur Verschäumung von Mitteln geeignet sind (mechanisch, hydraulisch etc.), auch solche für industrielle Anwendungen. Bevorzugt als Schaumspender sind Sprühcontainer. Überraschenderweise zeigte sich, daß sich die Mittel besonders zum Versprühen aus mechanischen Schaumspendern ohne Treibgas (z.B. die Produkte "Squeeze Foamer" und "F2 Finger Pump Foamer" der Firma Arispray International BV) eignen. Um eine gute Schaumbildung zu erreichen werden die Ölphasen und wässrigen Phasen bevorzugt vor dem Verschäumen mechanisch, bevorzugt durch einfaches Schütteln, miteinander vermischt.

Das nachfolgende Beispiel soll den Gegenstand der Erfindung näher erläutern, ohne ihn darauf einzuschränken. Bei allen Mengenangaben handelt es sich um Gewichtsprozent.

| Beispiel: Sonnenschutzschaum | | |
|---|---|---|
| A | ®Eusolex 2292 (Merck) (Ethylhexyl Methoxycinnamate) | 8,00 % |
| | ®Eusolex HMS (Merck) (Homosalate) | 8,00 % |
| | ®Eusolex 9020 (Merck) (Butyl Methoxydibenzoylmethan) | 4,00 % |
| | ®Eusolex 6300 (Merck) (4-Methylbenzyliden Camphor) | 4,00 % |
| | Isopropyl Palmitat | 2,00 % |
| | ®SilCare Silicone 41M15 (Clariant) (Caprylyl Methicon) | 1,00 % |
| | ®Eutanol G (Cognis) (Ethylhexyldodecanol) | 2,00 % |
| | | |
| B | ®Eusolex 232 (Merck) (Phenylbenzimidazole Sulfonic Acid) | 4,00 % |
| | Tromethamine | 2,21 % |
| | Glycerin | 7,00 % |
| | Panthenol | 0,50 % |
| | ®Hostapon CLG (Clariant) (Sodium Lauroyl Glutamate) | 1,00 % |
| | Wasser | ad 100 % |
| | Konservierungsmittel | q.s. |
| | Natriumchlorid | 1,00 % |
| | | |
| C | Parfüm | 0,30 % |

### Herstellung:

Die Phasen A, B und C wurden miteinander vermischt und anschließend wartete man ab bis die Phasentrennung in eine Ölphase und eine wässrige Phase erfolgt war.

### Verschäumung:

Die Verschäumung erfolgte mittels eines F2 Finger Pump Foamer der Firma Airspray International BV.
Dabei beobachtete man eine spontane Schaumbildung, die einen cremigen, feinporigen, leicht verteilbaren, stabilen Schaum erzeugte. Das kosmetische Mittel zog gut in die Haut ein und hinterließ ein angenehm pflegendes Hautgefühl.

## Patentansprüche

1. Verwendung von mehrphasigen Mitteln, enthaltend
a) mindestens eine Ölphase und
b) mindestens eine wässrige Phase,
zum Verschäumen aus Schaumspendern.

2. Verwendung nach Anspruch 1, **dadurch gekennzeichnet, dass** es sich bei den Mitteln um Zweiphasensysteme handelt.

3. Verwendung nach Anspruch 1 und/oder 2, **dadurch gekennzeichnet, dass** der Anteil der Ölphasen, bezogen auf die fertigen Mittel, 1 bis 50 Gew.-%, bevorzugt 3 bis 30 Gew.-%, beträgt.

4. Verwendung nach mindestens einem der Ansprüche 1 bis 3, **dadurch gekennzeichnet, dass** der Anteil der wässrigen Phasen beträgt, bezogen auf die fertigen Mittel, 50 bis 99 Gew.-%, bevorzugt 70 bis 97 Gew.-%, beträgt.

5. Verwendung nach mindestens einem der Ansprüche 1 bis 4, **dadurch gekennzeichnet, dass** es sich bei den Ölphasen um Guerbetalkohole, Fettsäureester, Ester von Fettalkoholen, Triglyceride, pflanzliche Öle, aliphatische und cycloaliphatische Kohlenwasserstoffe, Ether, Siliconöle und/oder Sonnenschutzverbindungen handelt.

6. Verwendung nach mindestens einem der Ansprüche 1 bis 5, **dadurch gekennzeichnet, dass** die wässrigen Phasen schaumverstärkende Tenside enthalten.

7. Verwendung nach Anspruch 6, **dadurch gekennzeichnet, dass** die schaumverstärkenden Tenside einen HLB-Wert von größer 18, bevorzugt größer 20, besonders bevorzugt von 20 bis 40, besitzen.

8. Verwendung nach Anspruch 6, **dadurch gekennzeichnet, dass** es sich bei den schaumverstärkenden Tensiden um Acylglutamate, (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate und/oder -glycinate handelt.

9. Verwendung nach Anspruch 8, **dadurch gekennzeichnet, dass** es sich bei den schaumverstärkenden Tensiden um Acylglutamate, bevorzugt Natrium Lauroylglutamat und/oder Natrium Cocoylglutamat, handelt.

10. Verwendung nach mindestens einem der Ansprüche 6 bis 9, **dadurch gekennzeichnet, dass** der Anteil der schaumverstärkenden Tenside in den wässrigen Phasen jeweils 0,1 bis 50 Gew.-%, bevorzugt 0,3 bis 20 Gew.-%, beträgt.

11. Verwendung nach mindestens einem der Ansprüche 1 bis 10, **dadurch gekennzeichnet, dass** die Viskositäten der Ölphasen und der wässrigen Phasen jeweils 1 bis 1000 mPas, bevorzugt 10 bis 500 mPas, betragen.

12. Verwendung nach mindestens einem der Ansprüche 1 bis 11, **dadurch gekennzeichnet, dass** die Mittel 0,01 bis 5 Gew.-% an Elektrolyten und/oder einund/oder mehrwertigen Alkoholen, jeweils bezogen auf die wässrigen Phasen, enthalten.

13. Verwendung nach Anspruch 12, **dadurch gekennzeichnet, dass** es sich bei den Elektrolyten um Alkalimetall- und/oder Erdalkalimetallsalze handelt.

14. Verwendung nach Anspruch 12, **dadurch gekennzeichnet, dass** es sich bei den Alkoholen um Propylenglykol, Isopropanol, Ethanol und/oder Polyethylenglykole handelt.

15. Verwendung nach mindestens einem der Ansprüche 1 bis 14, **dadurch gekennzeichnet, dass** es sich bei den Mitteln um kosmetische Mittel, bevorzugt Hautpflegemittel, handelt.

16. Verwendung nach Anspruch 15, **dadurch gekennzeichnet, dass** es sich bei den Mitteln um Sonnenschutzmittel, bevorzugt Sonnenschutzschäume, handelt.

17. Verwendung nach Anspruch 16, **dadurch gekennzeichnet, daß** die Ölphasen der Sonnenschutzmittel ganz oder teilweise aus Sonnenschutzverbindungen bestehen.

18. Verfahren zur Herstellung von Schaum, **dadurch gekennzeichnet, dass** man ein mehrphasiges Mittel, enthaltend
a) mindestens eine Ölphase und
b) mindestens eine wässrige Phase
mittels eines Schaumspenders verschäumt.

19. Schaumspender, enthaltend mindestens ein mehrphasiges Mittel, das
a) mindestens eine Ölphase und
b) mindestens eine wässrige Phase enthält.

20. Verwendung, Verfahren oder Schaumspender nach Anspruch 1, 18 oder 19, **dadurch gekennzeichnet, dass** es sich bei dem Schaumspender um einen Sprühcontainer handelt.

21. Verwendung, Verfahren oder Schaumspender nach Anspruch 1, 18 oder 19, **dadurch gekennzeichnet, dass** es sich bei dem Schaumspender um einen mechanischen Schaumspender ohne Treibgas handelt.
